(19) **Europäisches Patentamt / European Patent Office / Office européen des brevets**

(11) **EP 2 906 666 B1**

(12) **EUROPÄISCHE PATENTSCHRIFT**

(45) Veröffentlichungstag und Bekanntmachung des Hinweises auf die Patenterteilung:
**20.06.2018 Patentblatt 2018/25**

(21) Anmeldenummer: **13773816.7**

(22) Anmeldetag: **09.10.2013**

(51) Int Cl.:
*C07C 1/04* (2006.01)   *C07C 1/12* (2006.01)
*C10L 3/10* (2006.01)   *C10L 3/08* (2006.01)
*C10J 3/82* (2006.01)

(86) Internationale Anmeldenummer:
**PCT/EP2013/071095**

(87) Internationale Veröffentlichungsnummer:
**WO 2014/057004 (17.04.2014 Gazette 2014/16)**

(54) **VERFAHREN UND VORRICHTUNG ZUR ERZEUGUNG EINES METHANHALTIGEN ERDGASSUBSTITUTS**

PROCESS AND SYSTEM FOR PRODUCING A METHANE-CONTAINING NATURAL GAS SUBSTITUTE

PROCÉDÉ ET SYSTÈME POUR PRODUIRE SUBSTITUTE DU GAZ NATUREL CONTENANT METHANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.10.2012 DE 102012218526**

(43) Veröffentlichungstag der Anmeldung:
**19.08.2015 Patentblatt 2015/34**

(73) Patentinhaber: **Zentrum für Sonnenenergie- und Wasserstoff-Forschung Baden-Württemberg 70563 Stuttgart (DE)**

(72) Erfinder:
• STÜRMER, Bernd
  71088 Holzgerlingen (DE)
• SPECHT, Michael
  71111 Waldenbuch (DE)
• FRICK, Volkmar
  42249 Hisings Backa, Göteborg (SE)
• ZUBERBÜHLER, Ulrich
  70563 Stuttgart (DE)
• THALER, Sebastian
  71634 Ludwigsburg (DE)

(74) Vertreter: **Patentanwälte Ruff, Wilhelm, Beier, Dauster & Partner mbB Kronenstraße 30 70174 Stuttgart (DE)**

(56) Entgegenhaltungen:
**EP-A2- 1 227 141    WO-A1-2009/007061**

• STEFAN HEYNE ET AL: "Integration study for alternative methanation technologies for the production of synthetic natural gas from gasified biomass", 13TH CONFERENCE ON PROCESS INTEGRATION, MODELLING AND OPTIMISATION FOR ENERGY SAVING AND POLLUTION REDUCTION, 1. August 2010 (2010-08-01), XP055103849,
• JAN KOPYSCINSKI ET AL: "Production of synthetic natural gas (SNG) from coal and dry biomass - A technology review from 1950 to 2009", FUEL, Bd. 89, Nr. 8, 6. Februar 2010 (2010-02-06), Seiten 1763-1783, XP055000387, ISSN: 0016-2361, DOI: 10.1016/j.fuel.2010.01.027
• JENS SEHESTED ET AL: "Methanation of CO over Nickel: Mechanism and Kinetics at High $H_2$/CO Ratios +", THE JOURNAL OF PHYSICAL CHEMISTRY B, Bd. 109, Nr. 6, 1. Februar 2005 (2005-02-01), Seiten 2432-2438, XP055103675, ISSN: 1520-6106, DOI: 10.1021/jp040239s

## Beschreibung

Die Erfindung bezieht sich auf ein Verfahren und eine Vorrichtung zur Erzeugung eines methanhaltigen Erdgassubstituts aus einem kohlenoxidhaltigen Einsatzgas sowie auf ein mit einer derartigen Vorrichtung ausgerüstetes Energieversorgungssystem.

Unter Erdgassubstitut, abgekürzt SNG (Substitute Natural Gas), werden Gase verstanden, die zusätzlich zu oder anstelle von Erdgas verwendet werden können und dazu insbesondere in vorhandene Erdgas- bzw. Gasversorgungsnetze eingespeist werden können. Für eine solche Einspeisung müssen die Gase bestimmte Anforderungen erfüllen, die in zugehörigen Regeln und Vorschriften definiert bzw. standardisiert sind. So definieren beispielsweise die technischen Regeln der DVGW (Deutsche Vereinigung des Gas- und Wasserfaches e.V.) in den Arbeitsblättern G 260 "Gasbeschaffenheit" und G 262 "Nutzung von Gasen aus regenerativen Quellen in der öffentlichen Gasversorgung" die Anforderungen an Brenngase, die zur öffentlichen Gasversorgung genutzt werden. Dabei werden im Wesentlichen vier Gastypen unterschieden. Grundgase sind die in einem Versorgungsgebiet üblicherweise verteilten Gase. Konditionierungsgase sind Gase oder Gasgemische, die zur Einstellung brenntechnischer Kenndaten dem Grundgas bzw. dem einzuspeisenden Gas zugemischt werden. Zusatzgase sind Gase bzw. Gasgemische, die sich in Zusammensetzung und brenntechnischen Kenndaten wesentlich vom Grundgas unterscheiden und letzterem in begrenzter Menge zugesetzt werden können. Austauschgase sind Gase bzw. Gasgemische, die trotz einer vom Grundgas abweichenden Zusammensetzung und ggf. abweichenden Kenndaten bei gleichem Gasdruck und Geräteeinstellung ein gleichartiges Brennverhalten wie das Grundgas aufweisen und daher anstelle des Grundgases eingesetzt werden können. Austauschgas ist gegenüber Zusatzgas aufgrund der größeren Flexibilität im Hinblick auf die einspeisbaren Mengen von Vorteil, das einzuspeisende Gas muss dann jedoch strengere Qualitätsanforderungen erfüllen. Zu den wichtigsten brenntechnischen Kenndaten von Erdgas bzw. Erdgassubstitut gehört der sogenannte Wobbe-Index ($kWh/m^3_{Norm}$), der beispielsweise für Erdgas niedrigerer Qualität (Gruppe L-Gas) zwischen etwa 10,5 bis 13,0 und für Gas höherer Qualität (Gruppe H-Gas) zwischen etwa 12,8 und 15,7 liegt.

Eine in jüngster Zeit immer wichtiger werdende Quelle für Erdgassubstitut ist Biomasse, aus deren Umsetzung methanreiches Gas gewonnen werden kann, das als Erdgassubstitut eingesetzt werden kann. Die Biomasseumsetzung erfolgt typischerweise entweder durch eine thermochemische Vergasungsreaktion, die ein sogenanntes Synthesegas liefert, das die Hauptbestandteile Wasserstoff ($H_2$), Kohlenmonoxid (CO) und Kohlendioxid ($CO_2$) enthält und durch Methanisierung in $CH_4$ konvertiert werden kann, oder durch Biogasanlagen, die ein im Wesentlichen aus $CO_2$ und Methan ($CH_4$) bestehendes Produktgas liefern.

Es wurde bereits verschiedentlich vorgeschlagen, regenerativ erzeugte elektrische Energie, die z.B. in Form von Wind- und Sonnenenergie mit merklicher zeitlicher Fluktuation anfällt, bei der Erzeugung von Erdgassubstitut einzusetzen, um sie auf diese Weise als chemische Energie zu speichern. Dazu wird mit der erzeugten elektrischen Energie eine Wasserstofferzeugungseinrichtung, z.B. eine Wasserelektrolyseanlage, betrieben und der von dieser erzeugte Wasserstoff zusammen mit einem $CO_2$-haltigen Gas, das z.B. aus einer Biomasseumsetzung stammt, einer Methanisierungsreaktion unterzogen, aus deren Produktgas methanhaltiges Erdgassubstitut gewonnen werden kann. In der Offenlegungsschrift WO 2010/115983 A1 der Anmelderin wird ein Energieversorgungssystem offenbart, mit dem auf diese Weise ein Zusatzgas oder Austauschgas in einer variabel vorgebbaren, zur Einspeisung in ein Gasversorgungsnetz geeigneten Zusatz-/Austauschgasqualität bereitgestellt und genutzt wird.

In dem Zeitschriftenaufsatz M. Gassner et al., Integrated design of a gas separation system for the upgrade of crude SNG with membranes, Chemical Engineering and Processing 48 (2009), Seite 1391 bis 1404 werden ein Verfahren und eine Vorrichtung zur Erzeugung eines methanhaltigen Erdgassubstituts aus einer Methansynthese eines durch thermochemische Biomassevergasung gewonnenen Gases mit durch eine Elektrolyse gewonnenem Wasserstoff offenbart. Dabei wird das Produktgas aus der Methansynthese in einer einstufigen oder mehrstufigen Gasabtrenneinrichtung zum Erdgassubstitut aufbereitet, insbesondere hinsichtlich einer Abtrennung des im dortigen Produktgas der Methanisierungsreaktion enthaltenen $CO_2$. Das Permeatgas aus der Gasabtrennung wird einem Verbrennungsprozess zugeführt. Ähnlich wird in dem Zeitschriftenaufsatz M. Gassner und F. Maréchal, Combined mass and energy integration in process design at the example of membranebased gas separation systems, Computers and Chemical Engineering, 2010 eine Methansynthese eines durch thermochemische Vergasung von Biomasse gewonnenen Gases mit anschließender ein- oder mehrstufiger Gasabtrennung vorgeschlagen, um mit dem Retentatgas der Gasabtrennung methanreiches Erdgassubstitut bereitzustellen, wobei die Methansynthese in diesem Fall ohne extern zugeführten Wasserstoff durchgeführt wird und wasserstoffhaltiges Permeatgas aus der Gasabtrennung optional zur Eingangsseite der Methansynthese rückgeführt und dem Produktgas der thermochemischen Biomassevergasung zugeführt wird. Für die Zusammensetzung des Produktgases aus der thermochemischen Biomassevergasung als Einsatzgas für die Methanisierungsreaktion sind ein Gehalt von 27% bis 42% $H_2$, 18% bis 38% $CO_2$, 15% bis 28% CO und 9% bis 12% $CH_4$ angegeben (alle Angaben in Volumenprozent). Dies bedeutet, dass beim dort umgesetzten Einsatzgas der Wasserstoffanteil in einem stark unterstöchiometrischen Bereich bezogen auf den Gehalt an Kohlenoxiden, d.h. CO und $CO_2$, liegt.

Die Methanisierungsreaktion wird häufig als "chemische" Methanisierung durchgeführt und dazu katalytisch unterstützt, wobei verschiedene Katalysatormaterialien gängig sind, wie Nickel (Ni), Ruthenium (Ru), Eisen (Fe), Kobalt (Co), Rhodium (Rh), Palladium (Pd), Platin (Pt) und Iridium (Ir). Die Methanisierungskatalysatoren sind verschiedenen Deaktivierungsmechanismen unterworfen, insbesondere thermischer Sinterung, Kohlenstoffablagerung, Oxidation und Vergiftung durch Schwefelverbindungen, d.h. Sulfidbildung. Zur Vermeidung von thermischer Sinterung ist darauf zu achten, eine gewisse Katalysator-Maximaltemperatur im Betrieb nicht zu überschreiten. Kohlenstoffablagerungen bilden sich u.a. auf Basis der Boudouard-Reaktion und führen u.a. zu einem sogenannten Fouling der Katalysatoroberfläche, zum Blockieren von Poren und Hohlräumen des Katalysators und zur physikalischen Zerstörung des Katalysatorträgermaterials, für das üblicherweise ein Metalloxid, z.B. Aluminiumoxid, Kieselerde oder Kalkstein verwendet wird.

Alternativ kann die Methanisierungsreaktion als "biologische" Methanisierung durchgeführt werden, die eine Umsetzung von $CO_2$ mit $H_2$ durch Bakterien in einem entsprechenden biochemischen Reaktor bei Temperaturen von typischerweise weniger als 70°C beinhaltet.

In dem Aufsatz S. Heyne et al., Integration study for alternative methanation technologies for the production of synthetic natural gas from gasified biomass, Chem. Trans. 2010; 21; Seite 409 werden zwei unterschiedliche Methanisierungstechnolgien zur SNG-Gewinnung aus Biomasse vorgestellt, die vom adiabatischen Festbetttyp bzw. isothermischen Fluidbetttyp sind, wobei jeweils vorgeschlagen wird, zur Synthesegasmethanisierung Dampf in einem stöchiometrischen Anteil zuzugeben, um über die Wassergas-Shiftreaktion ein $H_2$/CO-Verhältnis von 3 zu erzielen.

Weitere herkömmliche Vorrichtungen und Verfahren zur SNG-Gewinnung sind in der Offenlegungsschrift EP 1 227 141 A2 und dem Übersichtsartikel J. Kopyscinski, Production of synthetic natural gas (SNG) from coal and dry biomass - A technological review from 1950 to 2009, Fuel 89 (2010), Seite 1763 beschrieben. Dazu wird in der EP 1 227 141 A2 eine SNG-Erzeugungsvorrichtung vorgeschlagen, die einen Bogenentladungsplasmareaktor mit einem Wassereinlass, einem Einlass für festen Kohlenstoff und einem Synthesegasauslass, eine Wasserzufuhrpumpe, die zugeführtes Wasser in Dampf wandelt, eine Bogenentladungs-Energieversorgung, um durch entsprechende Bogenentladung den Dampf mit dem festen Kohlenstoffmaterial zur Erzeugung von $H_2$ und CO enthaltendem Synthesegas zur Reaktion zu bringen, und einen Methanisierungsreaktor zur Umwandlung des Synthesegases in SNG umfasst. Mittels eines Flüssigkeit/Gas-Separators kann kondensiertes Wasser vom durch den Methanisierungsreaktor gelieferten SNG abgetrennt werden, wobei das abgetrennte Wasser über eine entsprechende Rückspeiseleitung zur Eingangsseite des Bogenentladungsplasmareaktors rückgeführt werden kann.

Der Erfindung liegt als technisches Problem die Bereitstellung eines Verfahrens und einer Vorrichtung der eingangs genannten Art sowie eines entsprechenden Energieversorgungssystems zugrunde, mit denen sich in gegenüber dem oben erläuterten Stand der Technik verbesserter Weise ein methanhaltiges Erdgassubstitut aus einem kohlenoxidhaltigen Einsatzgas gewinnen und nutzen lässt, beispielweise unter Einsatz von regenerativ erzeugter elektrischer Energie und/oder von Biomasse.

Die Erfindung löst dieses Problem durch die Bereitstellung eines Verfahrens mit den Merkmalen des Anspruchs 1 und einer Vorrichtung mit den Merkmalen des Anspruchs 7 sowie eines Energieversorgungssystems mit den Merkmalen des Anspruchs 10.

Beim erfindungsgemäßen Verfahren wird das Einsatzgas einer chemischen oder biologischen Methanisierungsreaktion unterzogen und das daraus gewonnene Produktgas mittels Gasabtrennung in ein methanhaltiges, das Erdgassubstitut lieferndes Retentatgas und ein wasserstoffhaltiges Permeatgas aufgeteilt. Wenigstens ein Teil des Permeatgases wird zum Einsatzgas rückgeführt und diesem unter Bildung eines entsprechenden Eduktgases für die Methanisierungsreaktion beigemischt. Charakteristischerweise wird für die Methanisierungsreaktion ein überstöchiometrischer Wasserstoffanteil im Eduktgas eingestellt.

Es zeigt sich, dass die Einstellung eines überstöchiometrischen Wasserstoffanteils im Eduktgas für die Methanisierungsreaktion wesentliche Vorteile gegenüber dem herkömmlichen Methanisierungsbetrieb mit unterstöchiometrischem oder allenfalls knapp stöchiometrischem Wasserstoffanteil hat, speziell hinsichtlich hohem Umsatzgrad der im Einsatzgas enthaltenen Kohlenoxide, insbesondere $CO_2$ und CO, und hinsichtlich Reduzierung von Deaktivierungseffekten eines im Methanisierungsreaktor üblicherweise enthaltenen Katalysatormaterials. So können durch den vergleichweise hohen Wasserstoffanteil im Eduktgas die Kohlenoxide praktisch vollständig in Methan umgesetzt werden und es kann ein Kühleffekt im Methanisierungsreaktor zur Vermeidung bzw. Abschwächung von Temperaturspitzen, den sogenannten Hot-Spots, bewirkt werden. Zudem wird ein inhärent sicherer Betrieb der Methanisierung bezüglich der verschiedenen Katalysator-Deaktivierungseffekte erzielt, wie Reduzierung bzw. Vermeidung von Kohlenstoffablagerungen sowie von Sulfidbildung, Oxidationsprozessen und thermischer Sinterung des Katalysatormaterials.

Der bei der Methanisierung nicht umgesetzte Wasserstoff wird als Bestandteil des Permeatgases durch die Gasabtrennung vom Retentatgas abgetrennt. Letzteres enthält das erzeugte Methan als Hauptbestandteil und liefert direkt oder nach weiterer Gasaufbereitung das gewünschte Erdgassubstitut. Je nach Erfordernis kann das so erzeugte Erdgassubstitut in Zusatzgasqualität oder Austauschgasqualität bereitgestellt werden, zum Beispiel zur Einspeisung in ein öffentliches Gasversorgungsnetz. Durch die Rückführung des Permeatgases kann der darin enthaltene, nicht umgesetzte Wasserstoff wieder der Methanisierungsreaktion zugeführt werden.

Die Methanisierung von CO und $CO_2$ mittels $H_2$ zu Methan ($CH_4$) und Wasser ($H_2O$) verläuft primär gemäß

den folgenden Methanisierungsreaktionen:

$$3H_2 + CO \rightleftarrows CH_4 + H_2O \quad \Delta H_R^0 = -206,4 \text{ kJ/mol} \qquad \text{(Gl. 1)}$$

$$4H_2 + CO_2 \rightleftarrows CH_4 + H_2O \quad \Delta H_R^0 = -164,9 \text{ kJ/mol.} \qquad \text{(Gl. 2)}$$

Die beiden Methanisierungsreaktionen sind durch die Shift-Reaktion

$$CO + H_2O \rightleftarrows H_2 + CO_2 \quad \Delta H_R^0 = -41,5 \text{ kJ/mol} \qquad \text{(Gl. 3)}$$

miteinander gekoppelt. Aufgrund der stark exothermen und volumenreduzierenden Hydrierung von CO und $CO_2$ laufen die Reaktionen bevorzugt bei niedrigen Temperaturen z.B. zwischen 180°C und 350°C und hohen Drücken ab. Die betragsmäßig große Reaktionsenthalpie ($\Delta H_R^0$) der CO-Methanisierung indiziert einen nahezu vollständigen Umsatz bei mäßigen Temperaturen, wodurch die Rückreaktion quasi vernachlässigt werden kann. Die $CO_2$-Methanisierung erfolgt überwiegend in zwei Schritten, dem CO-Retroshift und der anschließenden CO-Hydrierung, evtl. auch durch eine direkte Methanisierung

Der erfindungsgemäß überstöchiometrische Wasserstoffanteil im Eduktgas verschiebt die obigen Reaktionen jeweils in Richtung der wasserstofffreien Seite, d.h. die beiden Methanisierungsreaktionen nach rechts, und damit in Richtung erhöhtem Kohlenoxidumsatz. Gleichzeitig werden durch den vergleichsweise hohen Wasserstoffanteil im Eduktgas die nachfolgenden, katalysatorrelevanten Reaktionsgleichungen so verschoben, dass die für die Katalysatordeaktivierung primär verantwortlichen Effekte wie Kohlenstoffbildung und Metall-Sulfidbildung reduziert werden:

$$C + 2\,H_2 \rightarrow CH_4 \qquad \text{(Gl. 4)}$$

$$C + CO_2 \rightarrow 2CO \qquad \text{(Gl. 5)}$$

$$C + H_2O \rightarrow CO + H_2 \qquad \text{(Gl. 6)}$$

$$NiS + H_2 \rightarrow Ni + H_2S \qquad \text{(Gl. 7)}$$

$$Ni_3S_2 + 2\,H_2 \rightarrow 3\,Ni + 2\,H_2S \qquad \text{(Gl. 8)}$$

$$NiO + H_2 \rightarrow Ni + H_2O \qquad \text{(Gl. 9)}$$

Dabei werden die Reaktionen gemäß den obigen Gleichungen 5 und 6 von der Reaktion gemäß der obigen Gleichung 1 gefolgt. In den obigen Gleichungen 7 bis 9 wurde stellvertretend für andere einsetzbare, herkömmliche Katalysatormaterialien ein Nickelkatalysator betrachtet.

In einer Weiterbildung der Erfindung wird der Stöchiometriefaktor des Eduktgases, definiert als Verhältnis der Anteilsdifferenz von Wasserstoff und Kohlendioxid zur Anteilssumme von Kohlenmonoxid und Kohlendioxid, im Bereich größer drei und kleiner gleich fünfzehn gehalten. Bei dieser Definition hat der Stöchiometriefaktor bei stöchiometrischer Wasserstoffmenge den Wert 3, bei doppelt so hoher Wasserstoffmenge den Wert 7 und bei dreifach so hoher Wasserstoffmenge den Wert 11, jeweils bezogen auf den Fall der $CO_2$-Umsetzung und $CO_2$-Menge.

Eine Weiterbildung des erfindungsgemäßen Verfahrens gemäß Anspruch 3 umfasst verschiedene mögliche vorteilhafte Varianten für die Einstellung des überstöchiometrischen Wasserstoffanteils für die Methanisierungsreaktion. Dazu werden ein oder mehrere ausgewählte Verfahrensparameter gemessen und abhängig davon ein oder mehrere Verfahrensparameter geeignet eingestellt. Bei den Messgrößen kann es sich insbesondere um Druck, Temperatur und Volumenstrom bzw. Menge der beteiligten Gase und um die Ermittlung eines Druck- und/oder Temperaturprofils im Methanisierungsreaktor handeln. Bei den davon abhängig eingestellten Verfahrensparametern kann es sich insbesondere um die Menge an zugeführtem Einsatzgas und/oder Permeatgas, den Wasserstoffanteil im Einsatzgas, den Druck im Methanisierungsreaktor und/oder den für die Gasabtrennung gewählten Differenzdruck handeln.

So kann in einer dieser Varianten vorteilhaft die Beimischung des wasserstoffhaltigen Permeatgases zum Einsatzgas abhängig von einem vorgegebenen $CO_2/CO$-Gasvolumenstrom des Einsatzgases und einer vorgegebenen Druckdifferenz von Retentatgas und Permeatgas auf die Erzielung eines vorgegebenen $CO_2/CO$-Mindestumsatzes geregelt werden. Dabei gewährleistet die Rückführung einer ausreichenden Menge des wasserstoffhaltigen Permeatgases die Einhaltung eines überstöchiometrischen Wasserstoffanteils im Eduktgas.

Eine weitere Variante eignet sich in Ausgestaltung der Erfindung für Anwendungen, bei denen als Einsatzgas eine Mischung ein an Kohlenoxid, d.h. $CO_2$ und/oder CO, reichen Gases und eines Wasserstoffgases verwendet wird. Dies ist beispielsweise in Systemen der Fall, bei denen das $CO_2/CO$-reiche Gas aus einer Biomasseumsetzung stammt und ihm in einer Wasserelektrolyseanlage mit regenerativem elektrischem Strom erzeugter Wasserstoff beigemischt wird. Bei derartigen Anwendungen kann das Wasserstoffgas dem $CO_2/CO$-reichen Gas abhängig von der Menge und/oder der Zusammensetzung des $CO_2/CO$-reichen Gases und abhängig von der Menge und/oder der Zusammensetzung des rückgeführten Permeatgases zudosiert werden.

In einer Weiterbildung der Erfindung wird der Methanisierungsreaktor auf das Auftreten einer Katalysator-Deaktivierung und/oder einer Hot-Spot-Verschiebung überwacht, und bei erkannter Katalysator-Deaktivierung bzw. Hot-Spot-Verschiebung wird der Gasdruck im Methanisierungsreaktor und/oder ein Wasserdampfanteil im Eduktgas erhöht und/oder die Reaktor-Temperatur angepasst, d.h. je nach System und Anwendungsfall erhöht oder erniedrigt. In einer dieser Ausführungsvarianten wird der Druck und/oder die Temperatur im Methanisierungsreaktor an mehreren in Gasströmungsrichtung hintereinander angeordneten Messstellen gemessen, und anhand des entsprechenden Druck-/Temperaturprofils wird das Auftreten einer Hot-Spot-Verschiebung erkannt, die insbesondere durch eine zunehmende Katalysatordeaktivierung verursacht sein kann. Diesem Effekt kann dann durch die genannten Maßnahmen geeignet entgegengewirkt werden.

Eine erfindungsgemäße Vorrichtung zur Erzeugung eines methanhaltigen Erdgassubstituts aus einem kohlenoxidhaltigen Einsatzgas umfasst außer einem Methanisierungsreaktor und einer nachgeschalteten Gasabtrenneinrichtung eine Permeatgasrückführung zur Eingangsseite des Methanisierungsreaktors sowie eine Steuer- oder Regeleinrichtung zur gesteuerten oder geregelten Einstellung eines überstöchiometrischen Wasserstoffanteils im Eduktgas für die Methanisierungsreaktion. Eine solche Steuer- oder Regeleinrichtung gewährleistet die Einhaltung des gewünschten, überstöchiometrischen Wasserstoffanteils im Eduktgas. Insbesondere weist die erfindungsgemäße Vorrichtung die erforderlichen Mittel zur Durchführung des erfindungsgemäßen Verfahrens auf.

In Ausgestaltung der Erfindung weist die Vorrichtung dazu auch geeignete Sensormittel auf, mit denen Messinformationen für die Steuer- oder Regeleinrichtung gewonnen werden. Abhängig von den zugeführten Messinformationen greift die Steuer- oder Regeleinrichtung über entsprechende Stellglieder in den Verfahrensprozess derart ein, dass der Wasserstoffanteil im Eduktgas auf einem gewünschten überstöchiometrischen Wert bleibt.

Ein erfindungsgemäßes Energieversorgungssystem ist mit einer erfindungsgemäßen Vorrichtung zur Erzeugung eines methanhaltigen Erdgassubstituts ausgerüstet. Zusätzlich weist es eine Wasserstofferzeugungseinrichtung zur Erzeugung von Wasserstoff und eine Biomasseumsetzungsanlage oder eine andere, ein kohlenoxidhaltiges Gas liefernde $CO_2/CO$-Quelle auf. Diese liefern das Einsatzgas für die Methanisierung als ein Gemisch von kohlenoxidhaltigem Gas und Wasserstoffgas. Die Wasserstofferzeugungseinrichtung kann z.B. in Form einer Wasserelektrolyseanlage mit regenerativ erzeugter elektrischer Energie betrieben werden. Dies stellt dann ein System dar, mit dem in vorteilhafter Weise mit hohem Wirkungsgrad aus Biomasse gewonnene oder anderweitig bereitstehende Kohlenoxide und regenerativ erzeugte elektrische Energie zur Gewinnung von Erdgassubstitut eingesetzt werden können.

Vorteilhafte Ausführungsformen der Erfindung sind in den Zeichnungen dargestellt und werden nachfolgend beschrieben. Hierbei zeigen:

Fig. 1    ein schematisches Blockdiagramm eines Energieversorgungssystems mit einer Vorrichtung zur Erzeugung eines methanhaltigen Erdgassubstituts mit Methanisierungsreaktor und nachgeschalteter Gasabtrennung,

Fig. 2    ein Blockdiagramm einer exemplarischen, mehrstufigen Realisierung der Gasabtrenneinrichtung von Fig. 1,

Fig. 3    ein Kennliniendiagramm zur Veranschaulichung einer Hot-Spot-Verschiebung im Methanisierungsreaktor von Fig. 1 und

Fig. 4    ein Blockdiagramm eines weiteren Ausführungsbeispiels einer Vorrichtung zur Erzeugung eines methanhaltigen Erdgassubstituts.

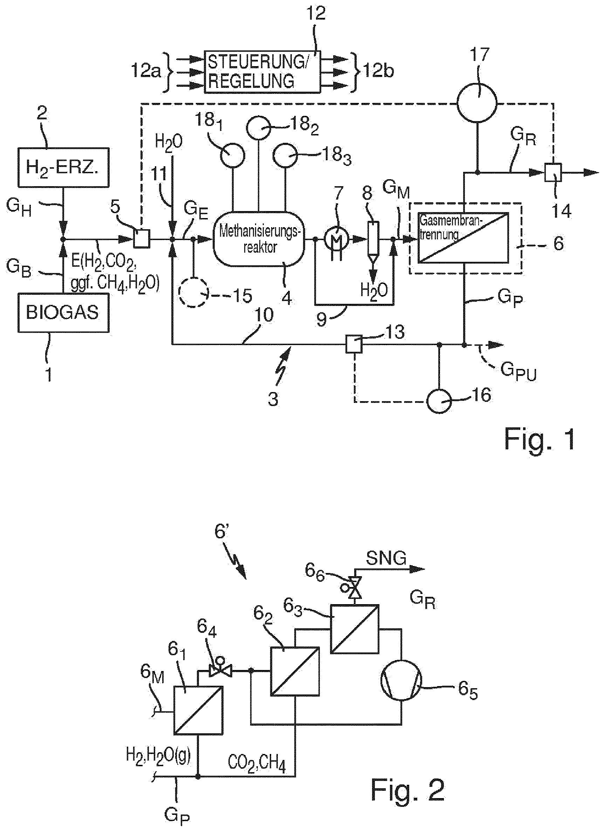

Ein erfindungsgemäßes Energieversorgungssystem ist mit seinen hier interessierenden Komponenten in Fig. 1 dargestellt. Das Energieversorgungssystem umfasst eine Biogasanlage 1, die in herkömmlicher Weise ein $CO_2$-reiches Produktgas $G_B$ liefert, und eine Wasserstofferzeugungseinrichtung 2, die Wasserstoff oder ein wasserstoffhaltiges Gas $G_H$ liefert und bei der es sich beispielsweise um eine Wasserelektrolyseanlage handeln kann, die mit regenerativ erzeugtem elektrischem Strom z.B. aus Windkraft- und/oder Photovoltaikanlagen betrieben wird. Diesen Komponenten ist als vorliegend primär interessierende Komponente eine Vorrichtung 3 zur Erzeugung eines methanhaltigen Erdgassubstituts aus einem Einsatzgas E nachgeschaltet, das durch Zudosierung des Wasserstoffgases $G_H$ aus der Wasserstofferzeugungseinrichtung 2 zum $CO_2$-reichen Produktgas $G_B$ der Biogasanlage 1 gebildet wird und somit $CO_2$ und $H_2$ als Hauptgasbestandteile neben etwaigen Minorkomponenten wie $CH_4$ und $H_2O$ beinhaltet. Auf die Vorrichtung 3 wird nachfolgend näher eingegangen, zu den übrigen Komponenten eines solchen Energieversorgungssystems kann für weitere Details z.B. auf die eingangs erwähnte WO 2010/115983 A1 verwiesen werden, deren Inhalt insoweit durch Bezugnahme hierin aufgenommen wird.

Zentraler Bestandteil der Erdgassubstitut-Erzeugungsvorrichtung 3 ist ein Methanisierungsreaktor 4 herkömmlichen Aufbaus, dem das Einsatzgas E über eine eingangsseitige Einsatzgas-Dosiereinrichtung 5 zudosiert werden kann. Der Reaktor 4 kann einstufig ausgeführt sein oder aus mehreren, z.B. in einer Kaskade angeordneten Reaktoreinheiten aufgebaut sein. Dem Methanisierungsreaktor 4 ist ausgangsseitig eine Gasabtrenneinrichtung 6 nachgeschaltet, der somit eingangsseitig ein Produktgas $G_M$ aus dem Methanisierungsreaktor 4 zugeführt wird. Dabei wird das Produktgas $G_M$ des Methanisierungsreaktors 4 über einen Wärmetauscher 7 und einen Wasserabscheider 8 geführt, die über eine Bypassleitung 9 umgangen werden können, bevor es in die Gasabtrenneinrichtung 6 eintritt.

Erfindungsgemäß wird der Methanisierungsreaktor 4 mit überstöchiometrischem Wasserstoffanteil betrieben, was zur Folge hat, dass im Produktgas $G_M$ des Methanisierungsreaktors 4 noch ein merklicher Wasserstoffanteil enthalten ist. Die Gasabtrenneinrichtung 6 ist dafür ausgelegt, diesen nicht umgesetzten Wasserstoff zusammen mit eventuell nicht umgesetztem Kohlenoxid, Wasser und etwaigen anderen Minorkomponenten als Permeatgas Gp abzutrennen und dadurch mit dem nichtabgetrennten Gas ein methanhaltiges Retentatgas $G_R$ zu liefern, das je nach Qualitätsanforderungen direkt oder mittels weiterer Gasaufbereitung als Erdgassubstitut verwendet werden kann.

Die Gasabtrenneinrichtung 6 kann von irgendeinem der hierfür bekannten herkömmlichen Bauarten sein, insbesondere als einstufige oder mehrstufige Abtrenneinheit vom Membrantyp. Hierzu sind je nach Anforderungen unterschiedliche Membrantypen im Stand der Technik bekannt, worauf verwiesen werden kann, wie solche vom Schlauchmembrantyp, z.B. Rohrmodule und Kapillar-/Hohlfasermodule, und vom Flachmembrantyp, z.B. Plattenmodule und Wickelmodule.

Fig. 2 zeigt stellvertretend für beliebige andere verwendbare, herkömmliche Gasabtrennmembraneinheiten eine dreistufige Gasabtrennmembraneinheit 6' mit drei bezüglich des Retentatgasstroms seriell hintereinander geschalteten Membranabtrennstufen $6_1$, $6_2$, $6_3$. Eine erste Membranabtrenneinheit $6_1$ trennt vorwiegend Wasserstoff und Wasserdampf aus dem zugeführten Produktgas $G_M$ des Methanisierungsreaktors ab. Das Retentatgas aus dieser ersten Stufe $6_1$ wird über ein Dosierventil $6_4$ einer zweiten Membranabtrenneinheit $6_2$ zugeführt, die speziell auf die Abtrennung von $CO_2$ und $CH_4$ ausgelegt ist. Das Permeatgas dieser mittleren Abtrennstufe $6_2$ wird dem Permeatgas aus der ersten Stufe $6_1$ zugegeben, was dann den gesamten Permeatgasstrom Gp dieser Gasmembranabtrenneinheit 6' bildet. Das Retentatgas der mittleren Stufe $6_2$ wird einer dritten Stufe $6_3$ zugeführt, in der eine Restabtrennung stattfindet, wobei das Permeatgas dieser letzten Stufe $6_3$ über einen Verdichter $6_5$ zur Eingangsseite der mittleren Abtrennstufe $6_2$ rückgeführt wird. Über ein weiteres Dosier-/Druckventil $6_6$ wird das Retentatgas der dritten Stufe $6_3$ als Erdgassubstitut, d.h. SNG, abgegeben. Es versteht sich, dass wie gesagt beliebige andere Varianten von Gasmembranabtrenneinrichtungen mit einer, zwei oder mehr seriellen Abtrennstufen verwendet werden können, wobei je nach Anwendungsfall unterschiedliche Rückführungen von Permeatgas einer späteren Stufe zur Eingangsseite einer früheren Stufe vorgesehen sein können.

Wieder auf Fig. 1 Bezug nehmend weist die Erdgassubstitut-Erzeugungsvorrichtung 3 eine Permeatgasrückführung 10 auf, durch die das Permeatgas Gp aus der Gasabtrenneinrichtung 6 zur Eingangsseite des Methanisierungsreaktors 4 rückgeführt und dort dem Einsatzgas E beigemischt wird, um ein entsprechendes Eduktgas $G_E$ zur Einspeisung in den Methanisierungsreaktor 4 zu bilden. Zusätzlich kann über eine zugehörige Wassereinspeiseleitung 11 Wasserdampf für das Eduktgas $G_E$ zudosiert werden. Nicht zur Rückführung benötigtes Permeatgas wird als sog. Purgegas $G_{PU}$ abgeführt.

Wie erwähnt, wird entsprechend einer erfindungsgemäßen Betriebsweise der Methanisierungsreaktor 4 mit überstöchiometrischem Wasserstoffanteil betrieben. Eine in Fig. 1 nur schematisch angedeutete Steuer- oder Regeleinrichtung 12 ist dazu vorgesehen, den Betrieb der Vorrichtung 3 so zu steuern bzw. zu regeln, dass für die Methanisierungsreaktion ein solcher überstöchiometrischer Wasserstoffanteil im Eduktgas $G_E$ bleibend beibehalten wird. Dazu gehören neben einer eigentlichen Steuer-/Regeleinheit die zugehörigen peripheren Komponenten wie Sensoren und Stellglieder. Dies ist dem Steuerungs-/Regelungsfachmann geläufig und bedarf hier keiner näheren Erläuterungen, so dass vorliegend nur auf Besonderheiten im Zusammenhang mit der Erdgassubstitut-Erzeugungsvorrichtung 3 eingegangen wird.

Zur Charakterisierung des überstöchiometrischen Wasserstoffanteils kann ein Stöchiometriefaktor S definiert werden durch

$$S = ([H_2]-[CO_2])/([CO]+[CO_2]),$$

d.h. der Stöchiometriefaktor ist in diesem Fall definiert als Verhältnis der Anteilsdifferenz von Wasserstoff und Kohlendioxid zur Anteilssumme von Kohlenmonoxid und Kohlendioxid im Eduktgas $G_E$. Mit dieser Definition entspricht bezogen auf die $CO_2$-Menge ein stöchiometrischer Wasserstoffanteil einem Stöchiometriefaktor S=3, für unterstöchiometrische Wasserstoffanteile liegt der Stöchiometriefaktor S unterhalb von drei, für überstöchiometrische Wasserstoffanteile liegt er oberhalb von drei. Vorzugsweise wird er, wie schon weiter oben erwähnt, auf Werte im überstöchiometrischen Bereich größer drei eingestellt, typisch bis zu einem Stöchiometriefaktor von fünfzehn. Wie aus der obigen Definition unmittelbar ersichtlich, kann der Stöchiometriefaktor S und damit der Wasserstoffanteil im Eduktgas $G_E$ durch Variation der zudosierten Wasserstoffmenge und/oder der zugeführten Menge an Kohlenoxiden des Einsatzgases E verändert werden. Die Zudosierung von Wasserstoff ist bei der Vorrichtung 3 sowohl anhand des Wasserstoffgases $G_H$ aus der Wasserstofferzeugungseinrichtung 2 als auch anhand des wasserstoffhaltigen, rückgeführten Permeatgases Gp variierbar.

In Fig. 1 sind einige vorliegend wichtige Stellglieder und Sensormittel explizit gezeigt, die mit der Steuer-/Regeleinrichtung 12 in Verbindung stehen, wobei in üblicher und daher hier nicht näher gezeigter Weise die Sensorausgangsinformationen einer Eingangsseite 12a der Steuer-/Regeleinrichtung 12 zugeführt werden und daraus abgeleitete Stellsignale von der Steuer-/Regeleinrichtung 12 über eine Ausgangsseite 12b an zugeordnete Stellglieder abgegeben werden.

So umfassen die Stellgliedmittel insbesondere die schon erwähnte Einsatzgas-Dosiereinrichtung 5 sowie eine Permeatgas-Dosiereinrichtung 13 und ein Retentatgas-Auslassventil 14. Die Sensormittel umfassen ein oder mehrere Eduktgas-Sensorelemente 15 zur Messung des Eduktgas-Volumenstroms und der Eduktgaszusammensetzung, d.h. der prozentualen Anteile der verschiedenen Eduktgaskomponenten, einen Permeatgas-Drucksensor 16 und ein oder mehrere Retentatgas-Sensorelemente 17 zur Messung des Drucks, der Temperatur, des Volumenstroms und/oder der Zusammensetzung des Retentatgases $G_R$. Mit gestrichelten Linien ist symbolisiert, welche Stelleingriffe die Steuer-/Regeleinrichtung 12 an welchen Stellgliedern aufgrund welcher Sensorinformationen vornimmt, wobei dies nur eine vereinfachende Illustration eines speziellen Beispiels ist und selbstverständlich andere Steuerungs-/Regelungsmaßnahmen realisierbar sind, wie dies der Fachmann versteht.

Zusätzlich sind im Methanisierungsreaktor 4 mehrere Sensorelemente $18_1$, $18_2$, $18_3$ an in Gasströmungsrichtung hintereinander liegenden Messstellen angeordnet, mit denen jeweils der Gasdruck und/oder die Temperatur an der betreffenden Messstelle erfasst werden kann. Mit dieser Sensorelementanordnung $18_1$, $18_2$, $18_3$ ist die Steuer-/Regeleinrichtung 12 in der Lage, den Methanisierungsreaktor 4 auf eine etwaige Hot-Spot-Verschiebung hin zu überwachen und die Betriebsführung hinsichtlich Methanisierungsreaktion und Gasabtrennung dadurch an Alterungserscheinungen von im Methanisierungsreaktor 4 verwendetem Katalysatormaterial anzupassen bzw. derartige Katalysatoralterungserscheinungen so gut wie möglich auszugleichen.

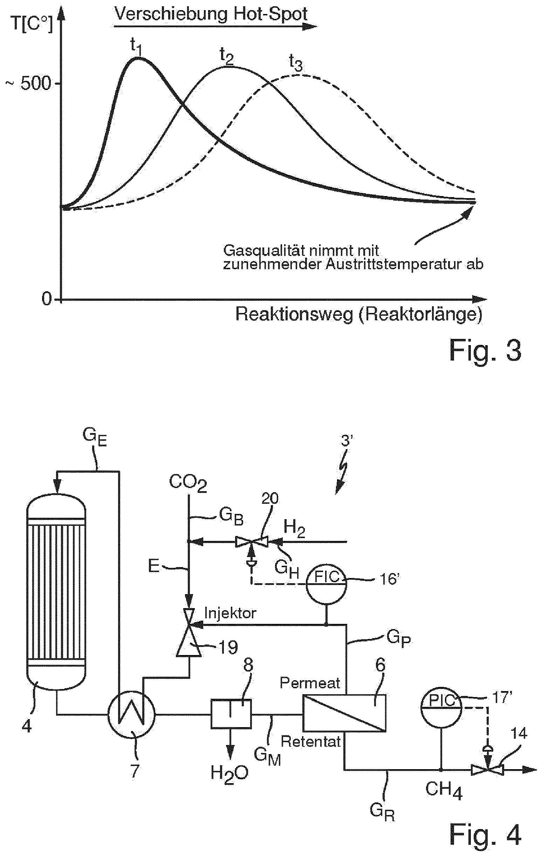

Dies wird nachstehend unter Bezugnahme auf Fig. 3 erläutert. Durch die Sensorelemente $18_1$, $18_2$, $18_3$ kann die Steuer-/Regeleinrichtung 12 ein Profil des Temperaturverlaufs im Methanisierungsreaktor 4 entlang des Reaktionsweges bzw. der Reaktorlänge ermitteln. Bekanntermaßen zeigt dieses Profil einen von der Eingangsseite aus zunächst stark ansteigenden und dann wieder allmählich abfallenden Temperaturverlauf mit einem als Hot-Spot bezeichneten Maximum aufgrund der exothermen Reaktionsenthalpie der im Methanisierungsreaktor 4 ablaufenden Reaktionen. Fig. 3 veranschaulicht ein entsprechendes Temperaturprofil zu drei verschiedenen Zeitpunkten, einem ersten Zeitpunkt $t_1$ mit noch nicht gealtertem Katalysatormaterial, einem Zeitpunkt $t_2$ mit bereits etwas gealtertem Katalysatormaterial und einem Zeitpunkt $t_3$ mit bereits stark gealtertem bzw. deaktiviertem Katalysatormaterial.

Wie aus Fig. 3 ersichtlich, verschiebt sich der Hot-Spot mit zunehmender Katalysatoralterung von der Reaktoreintrittsseite weg, und das Maximum nimmt etwas ab. Diese Hot-Spot-Verschiebung basiert auf den weiter oben erläuterten Katalysatordeaktivierungsmechanismen und kann von der Steuer-/Regeleinrichtung 12 aufgrund der betreffenden Sensorinformationen erkannt werden. Die Steuer-/Regeleinrichtung ist dann in der Lage, geeignete Gegenmaßnahmen einzuleiten, um den Effekten der Katalysatordeaktivierung entgegenzuwirken. So kann sie beispielsweise zur Aufrechterhaltung der Retentatgasqualität bei erkannter nachlassender Katalysatoraktivität den Druck im Methanisierungsreaktor 4 erhöhen und/oder die Druckdifferenz zwischen Retentatgas $G_R$ und Permeatgas Gp in der Gasabtrenneinrichtung 6 anheben, indem sie das Einsatzgas-Dosierventil 5 und/oder das Permeatgas-Dosierventil 13 und/oder das Retentatgas-Druckregelventil 14 entsprechend ansteuert. Dies erlaubt längere Betriebszeiten ohne Austausch des Katalysators, so dass Katalysatorbetriebszeiten von einigen Jahren ohne signifikante Verringerung der Retentatgasqualität möglich sind. Eine Katalysatordeaktivierung aufgrund von Kohlenstoffablagerungen auf dem Katalysator kann z.B. anhand des damit einhergehenden Druckverlustes im Methanisierungsreaktor 4 erkannt werden. Als steuernde bzw. regelnde Gegenmaßnahme kann der Wasserdampfanteil im Eduktgas $G_E$ erhöht werden, z.B. durch erhöhte Einspeisung über die Wasserzufuhrleitung 11.

Fig. 4 veranschaulicht eine Variante 3' der Vorrichtung 3 von Fig. 1 zur Erzeugung eines methanhaltigen Erdgassubstituts aus einem kohlenoxidhaltigen Einsatzgas, wobei identische und funktionell äquivalente Elemente der Übersichtlichkeit halber mit gleichen Bezugszeichen versehen sind und insoweit auf die obige Beschreibung zu Fig. 1 verwiesen werden kann. Bei der Vorrichtung 3' von Fig. 4 wird das wasserstoffreiche Permeatgas Gp über einen Injektor 19 angesaugt und dadurch dem Einsatzgas E beigemischt, das seinerseits aus einem $CO_2$-reichen Gas, wie z.B. dem Biogasanlagen-Produktgas $G_B$, und diesem zudosiertem Wasserstoffgas besteht. Das Retentatgas-Auslassventil 14 wird von einem Druckregler 17' der zugehörigen Steuer-/Regeleinrichtung gesteuert, und die Zudosierung von Wasserstoffgas zum $CO_2$-reichen Gas wird über ein Dosierventil 20 durch einen Gasvolumenstrom- bzw. Massenstromregler 16' der Steuer-/Regeleinrichtung abhängig vom gemessenen, angesaugten Permeatgas-Volumenstrom gesteuert.

Bei dieser Systemauslegung liegt selbst bei stöchiometrischer Zudosierung des Wasserstoffgases über das Dosierventil 20 zum CO$_2$-reichen Gas aufgrund des über den Injektor 19 zusätzlich zudosierten wasserstoffreichen Permeatgases Gp ein Eduktgas G$_E$ mit überstöchiometrischem Wasserstoffanteil vor. Der System-Prozessdruck kann in dieser Anlage über eine einfache Vor-Druckregelung konstant gehalten werden. Die H$_2$-Dosierung erfolgt im Verhältnis zur momentanen CO$_2$-Dosierung, die als Leitgröße dient. Dabei wird das Dosierventil 20 abhängig von der über den Permeatgasstrom Gp injizierten Wasserstoffmenge so gesteuert, dass insgesamt der gewünschte überstöchiometrische Wasserstoffanteil im Eduktgas G$_E$ eingestellt bleibt.

Vorstehend wurde der Methanisierungsreaktor 4 primär in einer Auslegung zur Durchführung einer chemischen Methanisierung erläutert, das heißt als chemischer Reaktor zur katalysatorgestützten Umsetzung der Edukte, insbesondere CO$_2$ mit H$_2$, in einem Temperaturbereich von vorzugsweise zwischen 180°C und 600°C. Alternativ kann der Methanisierungsreaktor 4 zur Durchführung einer biologischen Methanisierung als biochemischer Reaktor ausgeführt sein, der die Edukte, insbesondere CO$_2$ mit H$_2$, durch Bakterien in einem Temperaturbereich von typischerweise weniger 70°C umsetzt. Für beide Reaktortypen kann als Edukt alternativ auch Biogas, das heißt CH$_4$ und CO$_2$, zugeführt werden. Auch bei Durchführung der biologischen Methanisierung ist die Einstellung eines überstöchiometrischen Wasserstoffanteils im Eduktgas von Vorteil. Kohlendioxid reagiert nahezu quantitativ zu Methan. Aus dem Produktgas, bestehend aus dem Hauptanteil CH$_4$ und dem im Überschuss zugegebenen H$_2$, ist der H$_2$-Anteil soweit abzutrennen, dass das Gas die Kriterien für die Gasnetzeinspeisung erfüllt. Diese Abtrennaufgabe ist einfacher durchzuführen als die Abtrennung von CO$_2$ als nicht umgesetztes Eduktgas aus dem Produktgas. Im Eduktgas kann das Kohlendioxid in entsprechenden Ausführungsformen der Erfindung wenigstens teilweise durch Kohlenmonoxid ersetzt sein.

Wie die oben beschriebenen Ausführungsbeispiele deutlich machen, ermöglicht die Erfindung in vorteilhafter Weise die Erzeugung eines methanhaltigen Erdgassubstituts aus einem kohlenoxidhaltigen Einsatzgas mit einer chemischen oder biologischen Methanisierungsreaktion, für die ein überstöchiometrischer Wasserstoffanteil im Eduktgas mittels geeigneter Steuerung oder Regelung eingestellt wird. Damit lässt sich ein CO$_2$/CO-reiches Einsatzgas nahezu vollständig umsetzen, und die Methanisierung kann bezüglich einer Katalysatordeaktivierung in einem inhärent sicheren Betrieb betrieben werden, d.h. entsprechenden Deaktivierungseffekten kann durch geeignete Anpassung des Wasserstoffanteils im Eduktgas der Methanisierungsreaktion entgegengewirkt werden. Zudem erfüllt der überstöchiometrisch zugeführte Wasserstoffanteil, der insbesondere mit dem rückgeführten Permeatgas zudosiert werden kann, einen Kühleffekt im Methanisierungsreaktor zur Vermeidung bzw. Abschwächung von Hot-Spot-Erscheinungen.

**Patentansprüche**

1.  Verfahren zur Erzeugung eines methanhaltigen Erdgassubstituts aus einem kohlenoxidhaltigen Einsatzgas, bei dem

    - das Einsatzgas einer Methanisierungsreaktion unterzogen wird und daraus gewonnenes Produktgas durch eine Gasabtrennung in ein methanhaltiges, das Erdgassubstitut lieferndes Retentatgas und ein wasserstoffhaltiges Permeatgas aufgeteilt wird,
    - wobei wenigstens ein Teil des Permeatgases zum Einsatzgas rückgeführt und diesem unter Bildung eines entsprechenden Eduktgases für die Methanisierungsreaktion beigemischt wird, **dadurch gekennzeichnet, dass**
    - für die Methanisierungsreaktion ein überstöchiometrischer Wasserstoffanteil im Eduktgas eingestellt wird.

2.  Verfahren nach Anspruch 1, weiter **dadurch gekennzeichnet, dass** ein Stöchiometriefaktor (S) des Eduktgases, der als Verhältnis der Anteilsdifferenz von Wasserstoff und Kohlendioxid zur Anteilssumme von Kohlenmonoxid und Kohlendioxid definiert ist, im Bereich größer drei und kleiner gleich fünfzehn gehalten wird.

3.  Verfahren nach Anspruch 1 oder 2, weiter **dadurch gekennzeichnet, dass** einer oder mehrere der folgenden Parameter gemessen werden und abhängig davon eine Menge an zugeführtem Einsatzgas und/oder eine Menge an rückgeführtem Permeatgas und/oder ein Wasserstoffanteil des zugeführten Einsatzgases und/oder ein Druck in einem die Methanisierungsreaktion durchführenden Methanisierungsreaktor und/oder ein Gasabtrennungs-Differenzdruck eingestellt wird/werden: Volumenstrom des Eduktgases, Zusammensetzung des Eduktgases, Druck des Eduktgases, Volumenstrom des Einsatzgases, Zusammensetzung des Einsatzgases, Druck des Einsatzgases, Volumenstrom des Retentatgases, Zusammensetzung des Retentatgases, Druck des Retentatgases, Volumenstrom des Permeatgases, Zusammensetzung des Permeatgases, Druck des Permeatgases, Temperatur des Eduktgases, Temperatur des Einsatzgases, Temperatur des Retentatgases, Temperatur des Permeatgases, Druck und Temperatur im Methanisierungsreaktor an einer oder mehreren in Gasströmungsrichtung hintereinander angeordneten Druck- bzw. Temperaturmessstellen.

4. Verfahren nach Anspruch 3, weiter **dadurch gekennzeichnet, dass** abhängig von einem vorgegebenen $CO_2$/CO-Gasvolumenstrom des Einsatzgases und einer vorgegebenen Druckdifferenz von Retentatgas und Permeatgas die Permeatgasbeimischung zum Einsatzgas auf die Erzielung eines vorgegebenen $CO_2$/CO-Mindestumsatzes geregelt wird.

5. Verfahren nach Anspruch 3 oder 4, weiter **dadurch gekennzeichnet, dass** als Einsatzgas eine Mischung eines $CO_2$/CO-reichen Gases und eines $H_2$-Gases verwendet wird, das dem $CO_2$/COreichen Gas abhängig von der Menge und/oder Zusammensetzung des $CO_2$/CO-reichen Gases und von der Menge und/oder Zusammensetzung des rückgeführten und dem Einsatzgas beigemischten Permeatgases zudosiert wird.

6. Verfahren nach einem der Ansprüche 3 bis 5, weiter **dadurch gekennzeichnet, dass** der Methanisierungsreaktor auf das Auftreten einer Katalysator-Deaktivierung und/oder einer Hot-Spot-Verschiebung überwacht wird, und bei erkannter Katalysator-Deaktivierung bzw. Hot-Spot-Verschiebung der Gasdruck im Methanisierungsreaktor und/oder ein Wasserdampfanteil im Eduktgas erhöht und/oder die Reaktor-Temperatur angepasst wird.

7. Vorrichtung zur Erzeugung eines methanhaltigen Erdgassubstituts aus einem kohlenoxidhaltigen Einsatzgas, mit

- einem Methanisierungsreaktor (4) zur Gewinnung eines Produktgases aus einem zugeführten Eduktgas,
- einer Gasabtrenneinrichtung (6) zur Aufteilung des Produktgases in ein methanhaltiges, das Erdgassubstitut lieferndes Retentatgas und ein wasserstoffhaltiges Permeatgas und
- einer Permeatgasrückführung (10) zur Eingangsseite des Methanisierungsreaktors und zur dortigen Beimischung des Permeatgases zum Einsatzgas unter Bildung des Eduktgases, **gekennzeichnet durch**
- eine Steuer- oder Regeleinrichtung (12) zur gesteuerten oder geregelten Einstellung eines überstöchiometrischen Wasserstoffanteils im Eduktgas für die Methanisierungsreaktion.

8. Vorrichtung nach Anspruch 7, weiter **dadurch gekennzeichnet, dass** die Steuer- oder Regeleinrichtung dafür ausgelegt ist, einen Stöchiometriefaktor (S) des Eduktgases, der als Verhältnis der Anteilsdifferenz von Wasserstoff und Kohlendioxid zur Anteilssumme von Kohlenmonoxid und Kohlendioxid definiert ist, im Bereich größer drei und kleiner gleich fünfzehn zu halten.

9. Vorrichtung nach Anspruch 7 oder 8, weiter **gekennzeichnet durch** Sensormittel zur Gewinnung von Messinformationen für die Steuer- oder Regeleinrichtung, wobei die Sensormittel Mittel zur Erfassung wenigstens einer der Messgrößen Gasvolumenstrom, Gaszusammensetzung, Druck und Temperatur von wenigstens einem der Gase Eduktgas, Retentatgas, Permeatgas und Einsatzgas und/oder Mittel zur Druckmessung und/oder zur Temperaturmessung im Methanisierungsreaktor an einer oder mehreren in Gasströmungsrichtung hintereinander angeordneten Druck- bzw. Temperaturmessstellen umfassen.

10. Energieversorgungssystem mit

- einer Wasserstofferzeugungseinrichtung (2) zur Erzeugung von Wasserstoff und
- einer Biomasseumsetzungsanlage (1) oder einer anderen, ein kohlenoxidhaltiges Gas ($CO_2$/CO) liefernden $CO_2$/CO-Quelle, **gekennzeichnet durch**
- eine Vorrichtung (3) nach einem der Ansprüche 7 bis 9 zur Erzeugung eines methanhaltigen Erdgassubstituts, wobei der Vorrichtung Mittel zur Zuführung des von der Wasserstofferzeugungseinrichtung erzeugten Wasserstoffs und des kohlenoxidhaltigen Gases als Einsatzgas zugeordnet sind.

**Claims**

1. Method for generating a methane-containing substitute natural gas from a carbon oxide-containing input gas, wherein

- the input gas is subjected to a methanation reaction and product gas obtained therefrom is divided by gas separation into a methane-containing retentate gas, which yields the substitute natural gas, and a hydrogen-containing permeate gas,
- wherein at least a part of the permeate gas is returned to the input gas and admixed therein to form a corresponding reactant gas for the methanation reaction, **characterized in that**
- a superstoichiometric hydrogen fraction is established in the reactant gas for the methanation reaction.

2. Method according to claim 1, further **characterized in that** a stoichiometry factor (S) of the reactant gas, which is defined as the ratio of the difference in the proportions of hydrogen and of carbon dioxide to the sum total of the carbon monoxide and carbon dioxide proportions, is maintained in the range above three and not more than fifteen.

3. Method according to claim 1 or 2, further **characterized in that** one or more of the following parameters are measured and used to adjust an amount of supplied input gas and/or an amount of returned permeate gas and/or a hydrogen fraction of the supplied input gas and/or a pressure in a methanation reactor hosting the methanation reaction and/or a gas separation pressure difference: volume flow of reactant gas, composition of reactant gas, pressure of reactant gas, volume flow of input gas, composition of input gas, pressure of input gas, volume flow of retentate gas, composition of retentate gas, pressure of retentate gas, volume flow of permeate gas, composition of permeate gas, pressure of permeate gas, temperature of reactant gas, temperature of input gas, temperature of retentate gas, temperature of permeate gas, pressure and temperature in the methanation reactor at one or more pressure and temperature, respectively, measuring points arranged in succession in gas flow direction.

4. Method according to claim 3, further **characterized in that** the permeate gas admixture to the input gas is closed-loop controlled to achieving a specified $CO_2/CO$ minimum conversion according to a specified $CO_2/CO$ gas volume flow of the input gas and a specified pressure difference between retentate gas and permeate gas.

5. Method according to claim 3 or 4, further **characterized in that** the input gas used is a mixture of a $CO_2/CO$-rich gas and of an $H_2$ gas which is metered into the $CO_2/CO$-rich gas according to the amount and/or composition of the $CO_2/CO$-rich gas and the amount and/or composition of the returned permeate gas admixed to the input gas.

6. Method according to any of claims 3 to 5, further **characterized in that** the methanation reactor is monitored for a catalyst deactivation and/or a hot spot shift, and once catalyst deactivation and/or hot spot displacement has been detected, the gas pressure in the methanation reactor and/or a water vapor fraction in the reactant gas is increased and/or the reactor temperature is adjusted.

7. Apparatus for producing a methane-containing substitute natural gas from a carbon oxide-containing input gas, comprising

   - a methanation reactor (4) to obtain a product gas from a supplied reactant gas,
   - a gas separation device (6) for dividing the product gas into a methane-containing retentate gas, which yields the substitute natural gas, and a hydrogen-containing permeate gas, and
   - a permeate gas recycle (10) to the inlet side of the methanation reactor and to the admixture there of the permeate gas to the input gas to form the reactant gas, **characterized by**
   - an open or closed loop control device (12) for establishing a superstoichiometric hydrogen fraction in the reactant gas for the methanation reaction under open or closed loop control.

8. Apparatus according to claim 7, further **characterized in that** the open or closed loop control device is designed to maintain a stoichiometry factor (S) of the reactant gas, which is defined as the ratio of the difference in the proportions of hydrogen and of carbon dioxide to the sum total of the carbon monoxide and carbon dioxide proportions, in the range above three and not more than fifteen.

9. Apparatus according to claim 7 or 8, further **characterized by** a sensor device to generate measured data for the open or closed loop control device, wherein the sensor device includes means for capturing at least one of the measuring entities gas volume flow, gas composition, pressure and temperature values measured for one or more of reactant gas, retentate gas, permeate gas and input gas, and/or means for pressure measurement and/or for temperature measurement in the methanation reactor at one or more pressure and temperature, respectively, measuring points arranged in succession in the gas flow direction.

10. Energy supply system, comprising

   - a hydrogen generating device (2) for generating hydrogen and
   - a biomass conversion plant (1) or some other $CO_2/CO$ source delivering a carbon oxide-containing gas ($CO_2/CO$),

   **characterized by**

- an apparatus (3) according to any of claims 7 to 9 for producing a methane-containing substitute natural gas, wherein means for supplying the hydrogen generated by the hydrogen generating device and the carbon oxide-containing gas as input gas are assigned to the apparatus.

**Revendications**

1. Procédé pour générer un substitut de gaz naturel contenant du méthane à partir d'un gaz initial contenant de l'oxyde de carbone, dans lequel

   - le gaz initial est soumis à une réaction de méthanisation et le gaz produit obtenu à partir de celle-ci est réparti par une séparation des gaz en un gaz de retentat contenant du méthane, fournissant le substitut du gaz naturel, et un gaz de perméat contenant de l'hydrogène,
   - au moins une partie du gaz de perméat étant recyclée vers le gaz initial et mélangée à celui-ci avec formation d'un gaz de départ correspondant pour la réaction de méthanisation,
   **caractérisé en ce que**
   - une proportion d'hydrogène supérieure à la quantité stoechiométrique est réglée dans le gaz de départ pour la réaction de méthanisation.

2. Procédé selon la revendication 1, **caractérisé en outre en ce qu'**un facteur stoechiométrique (S) du gaz de départ, qui est défini comme le rapport de la différence de proportions d'hydrogène et de dioxyde de carbone à la somme des proportions de monoxyde de carbone et de dioxyde de carbone, est maintenu dans une plage supérieure à 3 et inférieure à 15.

3. Procédé selon la revendication 1 ou 2, **caractérisé en outre en ce qu'**un ou plusieurs des paramètres suivants sont mesurés et, en fonction de celui-ci/ceux-ci, une quantité de gaz initial alimenté et/ou une quantité de gaz de perméat recyclé et/ou une proportion d'hydrogène du gaz initial alimenté et/ou une pression dans un réacteur de méthanisation effectuant la réaction de méthanisation et/ou une pression différentielle de la séparation du gaz est/sont réglée(s) : flux volumique du gaz de départ, composition du gaz de départ, pression du gaz de départ, flux volumique du gaz initial, composition du gaz initial, pression du gaz initial, flux volumique du gaz de retentat, composition du gaz de retentat, pression du gaz de retentat, flux volumique du gaz de perméat, composition du gaz de perméat, pression du gaz de perméat, température du gaz de départ, température du gaz initial, température du gaz de retentat, température du gaz de perméat, pression et température dans le réacteur de méthanisation au niveau d'un ou de plusieurs site(s) de mesure de la pression ou de la température disposés les uns derrière les autres dans le sens d'écoulement du gaz.

4. Procédé selon la revendication 3, **caractérisé en outre en ce que**, en fonction d'un flux volumique de $CO_2/CO$ gazeux défini au préalable du gaz initial et d'une différence de pression définie au préalable du gaz de retentat et du gaz de perméat, le mélange supplémentaire de gaz de perméat au gaz initial est régulé en fonction de l'obtention d'une conversion minimale $CO_2/CO$ définie au préalable.

5. Procédé selon la revendication 3 ou 4, **caractérisé en outre en ce qu'**on utilise, comme gaz initial, un mélange d'un gaz riche en $CO_2/CO$ et de $H_2$ gazeux, qui est dosé dans le gaz riche en $CO_2/CO$ en fonction de la quantité et/ou de la composition du gaz riche en $CO_2/CO$ et de la quantité et/ou de la composition du gaz de perméat recyclé et mélangé au gaz initial.

6. Procédé selon l'une quelconque des revendications 3 à 5, **caractérisé en outre en ce que** le réacteur de méthanisation est surveillé en ce qui concerne la survenue d'une désactivation du catalyseur et/ou d'un déplacement d'un point chaud et, lorsqu'une désactivation du catalyseur ou, selon le cas, un déplacement de point chaud est détecté(e), la pression gazeuse dans le réacteur de méthanisation et/ou une proportion de vapeur d'eau dans le gaz de départ est/sont augmentée(s) et/ou la température du réacteur est adaptée.

7. Dispositif pour générer un substitut de gaz naturel contenant du méthane à partir d'un gaz initial contenant de l'oxyde de carbone, présentant

   - un réacteur de méthanisation (4) pour générer un gaz produit à partir d'un gaz de départ alimenté,
   - un dispositif (6) de séparation de gaz pour répartir le gaz produit en un gaz de retentat contenant du méthane, fournissant le substitut du gaz naturel, et un gaz de perméat contenant de l'hydrogène, et

- un recyclage (10) de gaz de perméat vers le côté entrée du réacteur de méthanisation et vers le mélange en cet endroit du gaz de perméat au gaz initial avec formation du gaz de départ,

**caractérisé par**

- un dispositif (12) de commande ou de régulation pour le réglage commandé ou régulé d'une proportion d'hydrogène supérieure à la quantité stoechiométrique dans le gaz de départ pour la réaction de méthanisation.

**8.** Dispositif selon la revendication 7, **caractérisé en outre en ce que** le dispositif de commande ou de régulation est conçu pour maintenir un facteur stoechiométrique (S) du gaz de départ, qui est défini comme le rapport de la différence de proportions d'hydrogène et de dioxyde de carbone à la somme des proportions de monoxyde de carbone et de dioxyde de carbone, dans une plage supérieure à 3 et inférieure à 15.

**9.** Dispositif selon la revendication 7 ou 8, **caractérisé en outre par** des moyens de capteur pour obtenir des informations de mesure pour le dispositif de commande ou de régulation, les moyens de capteur comprenant des moyens pour détecter au moins une des dimensions de mesure flux volumique gazeux, composition du gaz, pression et température d'au moins un des gaz gaz de départ, gaz de retentat, gaz de perméat et gaz initial et/ou des moyens pour la mesure de la pression et/ou pour la mesure de la température dans le réacteur de méthanisation en un ou plusieurs site(s) de mesure de la pression ou de la température disposés les uns derrière les autres dans le sens d'écoulement du gaz.

**10.** Système d'alimentation en énergie présentant

- un dispositif (2) de génération d'hydrogène destiné à produire de l'hydrogène et
- une installation (1) de conversion de biomasse ou d'une autre source de $CO_2/CO$ fournissant un gaz contenant de l'oxyde de carbone ($CO_2/CO$),

**caractérisé par**

- un dispositif (3) selon l'une quelconque des revendications 7 à 9 pour la production d'un substitut de gaz naturel contenant du méthane, des moyens pour alimenter l'hydrogène généré dans le dispositif de génération d'hydrogène et le gaz contenant de l'oxyde de carbone comme gaz initial étant associés au dispositif.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010115983 A1

- EP 1227141 A2

**In der Beschreibung aufgeführte Nicht-Patentliteratur**

- **M. GASSNER et al.** Integrated design of a gas separation system for the upgrade of crude SNG with membranes. *Chemical Engineering and Processing,* 2009, vol. 48, 1391-1404
- **M. GASSNER ; F. MARÉCHAL.** Combined mass and energy integration in process design at the example of membranebased gas separation systems. *Computers and Chemical Engineering,* 2010

- **S. HEYNE et al.** Integration study for alternative methanation technologies for the production of synthetic natural gas from gasified biomass. *Chem. Trans.,* 2010, vol. 21, 409
- **J. KOPYSCINSKI.** Production of synthetic natural gas (SNG) from coal and dry biomass - A technological review from 1950 to 2009. *Fuel,* 2010, vol. 89, 1763